(19) Europäisches Patentamt / European Patent Office / Office européen des brevets

(11) **EP 3 255 997 B1**

(12) **EUROPEAN PATENT SPECIFICATION**

(45) Date of publication and mention
of the grant of the patent:
**04.12.2024 Bulletin 2024/49**

(21) Application number: **16703796.9**

(22) Date of filing: **10.02.2016**

(51) International Patent Classification (IPC):
**A23C 9/12** *(2006.01)*    **A23C 19/032** *(2006.01)*
**A23C 19/04** *(2006.01)*    **A23C 19/068** *(2006.01)*
**C12N 9/64** *(2006.01)*

(52) Cooperative Patent Classification (CPC):
**A23C 19/04; A23C 19/0326; A23C 19/0682;
C12N 9/6483; C12Y 304/23004**

(86) International application number:
**PCT/EP2016/052842**

(87) International publication number:
**WO 2016/128476 (18.08.2016 Gazette 2016/33)**

(54) **BLENDS OF CHYMOSINS WITH IMPROVED MILK-CLOTTING PROPERTIES**

MISCHUNGEN AUS CHYMOSINEN MIT VERBESSERTEN
MILCHGERINNUNGSEIGENSCHAFTEN

MÉLANGES DE CHYMOSINES AVEC DES PROPRIÉTÉS DE CAILLAGE DU LAIT AMÉLIORÉES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.02.2015 EP 15154513**

(43) Date of publication of application:
**20.12.2017 Bulletin 2017/51**

(73) Proprietor: **Chr. Hansen A/S
2970 Hoersholm (DK)**

(72) Inventors:
• **FAIVELEY, Marc
33140 Villenave d'Ornon (FR)**
• **BROCHERET, Sylvain
75017 Paris (FR)**
• **POIGNAND, Jean-Paul
25000 Besançon (FR)**
• **DE LAMOTTE, Stephane
53200 Fromentières (FR)**
• **ROUSTEL, Sebastien
39210 Chateau-Chalon (FR)**

(56) References cited:
**WO-A1-2008/098973    WO-A2-02/36752
FR-A1- 2 494 086**

• **MINISTÈRE DE ET AL: "Décrets, arrêtés,
circulaires Textes généraux", MÉDECINE DES
MALADIES MÉTABOLIQUES, 20 September 2008
(2008-09-20), pages S67 - S69, XP055267248,
Retrieved from the Internet
<URL:https://www.legifrance.gouv.fr/jopdf//jopd
f/2008/0921/joe_20080921_0011.pdf> [retrieved
on 20160420], DOI:
10.1016/S1957-2557(10)70673-8**

Remarks:
The complete document including Reference
Table(s) and the Sequence Listing(s) can be
downloaded from the EPO website

## Description

### FIELD OF THE INVENTION

The current invention relates to blends of coagulants and/or mixtures thereof with improved cheese making properties.

The invention is defined by the appended claims.

### BACKGROUND ART

Enzymatic coagulation of milk by milk-clotting enzymes, such as chymosin and pepsin, is one of the most important processes in the manufacture of cheeses. Enzymatic milk coagulation is a two-phase process: a first phase where a proteolytic enzyme, chymosin or pepsin, attacks κ-casein, resulting in a metastable state of the casein micelle structure and a second phase, where the milk subsequently coagulates and forms a coagulum.

Chymosin (EC 3.4.23.4) and pepsin (EC 3.4.23.1), the milk clotting enzymes of the mammalian stomach, are aspartic proteases belonging to a broad class of peptidases.

When produced in the gastric mucosal cells, chymosin and pepsin occur as enzymatically inactive pre-prochymosin and pre-pepsinogen, respectively. When chymosin is excreted, an N-terminal peptide fragment, the pre-fragment (signal peptide) is cleaved off to give prochymosin including a pro-fragment. Prochymosin is a substantially inactive form of the enzyme which, however, becomes activated under acidic conditions to the active chymosin by autocatalytic removal of the pro-fragment. This activation occurs in vivo in the gastric lumen under appropriate pH conditions or in vitro under acidic conditions.

The structural and functional characteristics of bovine, i.e. *Bos taurus,* pre-prochymosin, prochymosin and chymosin have been studied extensively. The pre-part of the bovine pre-prochymosin molecule comprises 16 aa residues and the pro-part of the corresponding prochymosin has a length of 42 aa residues. The active bovine chymosin comprises 323 aa is a mixture of two forms, A and B, both of which are active.

Chymosin is produced naturally in mammalian species such as bovines, camels, caprines, buffaloes, sheep, pigs, humans, monkeys and rats.

Bovine chymosin has for a number of years been commercially available to the dairy industry.

WO02/36752A2 (Chr. Hansen) describes recombinant production of camel chymosin.

WO2013/174840A1 (Chr. Hansen) describes mutants/variants of bovine and camel chymosin.

WO2013/164479A2 (DSM) describes mutants of bovine chymosin.

The references listed immediately below may in the present context be seen as references describing mutants of chymosin:

- Suzuki et al: Site directed mutagenesis reveals functional contribution of Thr218, Lys220 and Asp304 in chymosin, Protein Engineering, vol. 4, January 1990, pages 69-71;
- Suzuki et al: Alteration of catalytic properties of chymosin by site-directed mutagenesis, Protein Engineering, vol. 2, May 1989, pages 563-569;
- van den Brink et al: Increased production of chymosin by glycosylation, Journal of biotechnology, vol. 125, September 2006, pages 304-310;
- Pitts et al: Expression and characterisation of chymosin pH optima mutants produced in Tricoderma reesei, Journal of biotechnology, vol. 28, March 1993, pages 69-83;
- M.G. Williams et al: Mutagenesis, biochemical characterization and X-ray structural analysis of point mutants of bovine chymosin, Protein engineering design and selection, vol. 10, September 1997, pages 991-997;
- Strop et al: Engineering enzyme subsite specificity: preparation, kinetic characterization, and x-ray analysis at 2.0 ANG resolution of Val111phe site mutated calf chymosin, Biochemistry, vol. 29, October 1990, pages 9863-9871;
- Supannee et al: Site-specific mutations of calf chymosin B which influence milk-clotting activity, Food Chemistry, vol. 62, June 1998, pages 133-139;
- Zhang et al: Functional implications of disulfide bond, Cys45-Cys50, in recombinant prochymosin, Biochimica et biophysica acta, vol. 1343, December 1997, pages 278-286.

The prior art references mentioned above focus on the molecular structure and its impact on the specificity or performance of the chymosins.

None of the references disclose the combined effects of different chymosins with different properties and from different origins.

## SUMMARY OF THE INVENTION

The inventors of present invention have discovered that specific coagulants and/or mixtures thereof may be applied in controlling the bacterial acidification rate and acidification end-point during cheese formation.

Furthermore, the inventors have found that present invention allow a feasible control of the structure development of the soft cheese during ripening and storage by providing specific blends of chymosins.

As discussed in working Examples herein - the present inventors have also identified a number of coagulant blends showing commercially attractive traits such as e.g. accelerated curd firmness development that exceed the expected performance.

Based on a comparative analysis of the coagulant blends, the present inventors identified a number of blends that are herein important in the sense that by making a blend one may get an improved and superior coagulant performance.

## DEFINITIONS

All definitions of herein relevant terms are in accordance of what would be understood by the skilled person in relation to the herein relevant technical context.

The term "coagulant" relates to an enzyme used to coagulate milk in a cheese making process. For the sake of completeness, chymosin is to be considered as a coagulant.

The term "high C/P coagulant" or "a high C/P ratio coagulant" relates to a coagulant, such as e.g. a chymosin having a C/P>4.5 such as e.g. C/P>5, such as e.g. C/P>6, such as e.g. C/P>7 or a C/P between 6 and 12. Commercially available examples of a high C/P coagulants comprise ChyMax M or ChyMax M1000 (Chr. Hansen A/S) and MaxirenXDS (DSM).

The term "low C/P coagulant" or "low C/P ratio coagulant" relates to a coagulant such as e.g. a chymosin having a C/P<4,5, such as e.g. a C/P<4, such as e.g. a C/P<3, such as e.g. a C/P<2, such as e.g. a C/P<1.5, such as e.g. a C/P<1, such as e.g. a C/P<0.5 or C/P<0.1. Commercially available examples of low C/P coagulants comprise Hannilase L®, Hannilase XP®, Thermolase® and Naturen® all available from Chr. Hansen A/S.

The term "coagulant blend" relates to blends of enzymes used to coagulate milk, e.g. in a cheese making process.

The term "chymosin" relates to an enzyme of the EC 3.4.23.4 class. Chymosin has a high specificity and it clots milk by cleavage of a single 105-Ser-Phe-|-Met-Ala-108 bond in kappa-chain of casein. An alternative name used in the art is rennin.

The term "chymosin activity" relates to chymosin activity of a chymosin enzyme as understood by the skilled person in the present context. The skilled person knows how to determine herein relevant chymosin activity.

In working Example 2 herein is provided an Example of a standard method to determine specific chymosin activity - alternatively termed clotting activity or milk clotting activity. As an example the clotting activity may be determined using the REMCAT method, which is the standard method developed by the International Dairy Federation (IDF method).

The term "isolated variant" means a variant that is modified by the hand of man.

The term "mature polypeptide" means a peptide in its final form following translation and any post-translational modifications, such as N terminal processing, C terminal truncation, glycosylation, phosphorylation, etc. In the present context may a herein relevant mature chymosin polypeptide be seen as the active chymosin polypeptide sequence - i.e. without the pre-part and/or pro-part sequences.

The term "parent" or "parent polypeptide having chymosin activity" means a polypeptide to which an alteration is made to produce the enzyme variants of the present invention. The parent may be a naturally occurring (wild-type) polypeptide or a variant thereof.

The term "Sequence Identity" relates to the relatedness between two amino acid sequences or between two nucleotide sequences and may calculated according to the methods available to the person skilled in the art.

The term "variant" means a peptide having chymosin activity comprising an alteration, i.e., a substitution, insertion, and/or deletion, at one or more (several) positions. A substitution means a replacement of an amino acid occupying a position with a different amino acid; a deletion means removal of an amino acid occupying a position; and an insertion means adding 1-3 amino acids adjacent to an amino acid occupying a position.

The term "wild-type" chymosin peptide means a chymosin expressed by a naturally occurring organism, such as e.g. a mammalian (e.g. camel or bovine) found in nature.

The term "coagulants with different origins" relates to coagulants derived from different organisms or alternatively by means of genetic modification. Hence a bovine wild-type chymosin and a genetically modified bovine chymosin are in the context of present invention to be considered as coagulants with different origins.

**DRAWINGS**

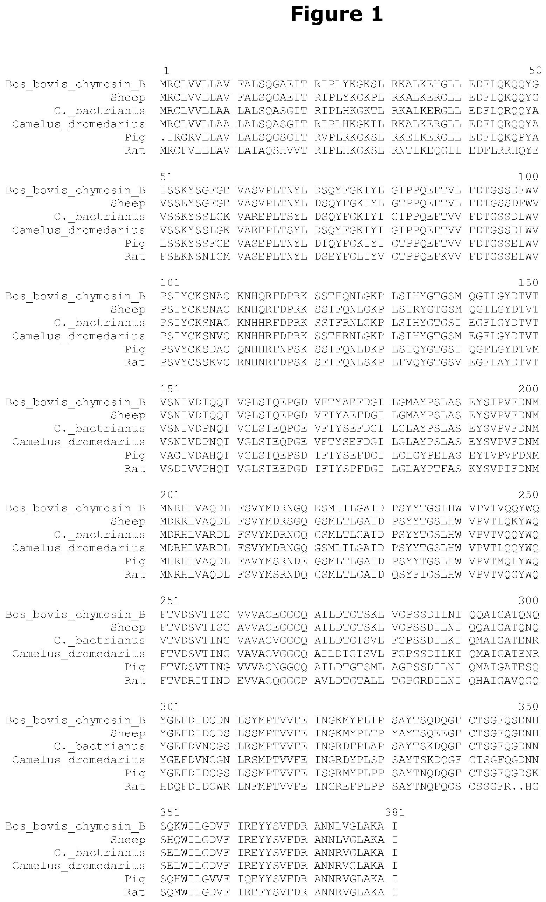

<u>Figure 1</u>: An alignment of herein relevant different chymosin sequences. The shown "Bos_bovis_chymosin_B" is bovine chymosin of SEQ ID NO: 1 herein and the shown "Camelus_dromedarius" is camel chymosin of SEQ ID NO: 2 herein. Using bovine chymosin of SEQ ID NO: 1 as reference sequence as described herein is can e.g. be seen that bovine chymosin has "V" in position 10 and camel chymosin has "A" in the same position 10. It may e.g. also be seen that bovine/Rat have "Q" in position 352 and Camel/C._bactrianus have "E" in the same position 352.

In relation to the chymosin sequences shown in figure 1 - sheep has 94.5% sequence identity with bovine SEQ ID NO: 1; C._bactrianus has 83.2% sequence identity with bovine SEQ ID NO: 1; Camelus_dromedarius (camel chymosin of SEQ ID NO: 2) has 84% sequence identity with bovine SEQ ID NO: 1; pig has 80.3% sequence identity with bovine SEQ ID NO: 1 and rat has 71.9% sequence with bovine identity SEQ ID NO: 1.

As understood by the skilled person in the present context - herein relevant sequence identity percentages of mature polypeptide sequences of e.g. sheep, C._bactrianus, camel, pig or rat chymosin with the mature polypeptide of SEQ ID NO: 1 (bovine chymosin - i.e. amino acid positions 59 to 381 of SEQ ID NO: 1) are relatively similar to above mentioned sequence identity percentages.

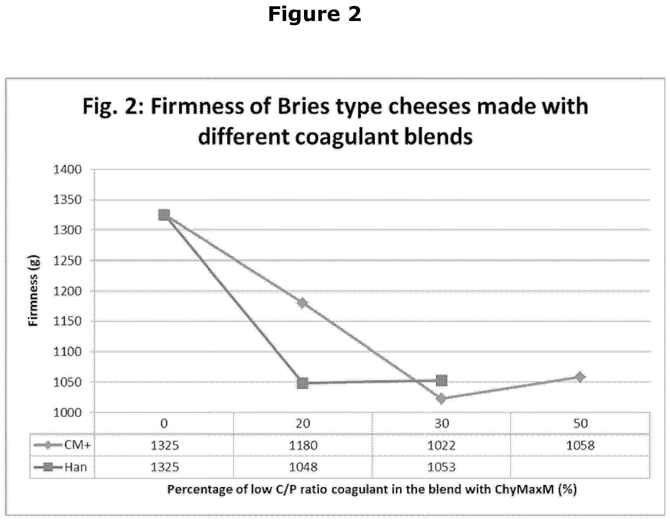

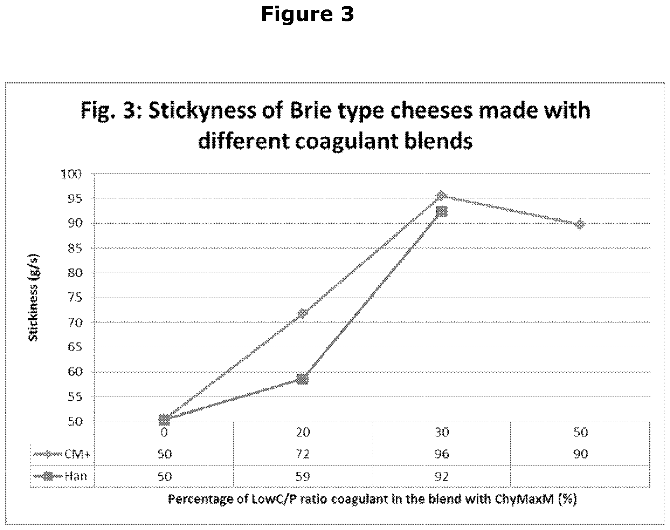

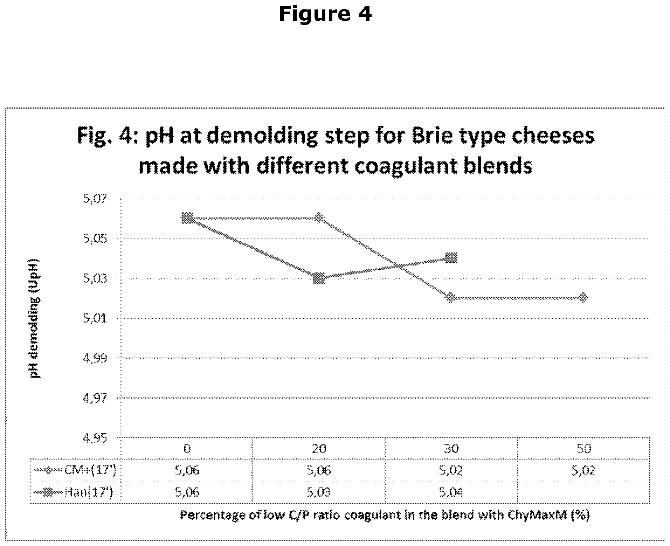

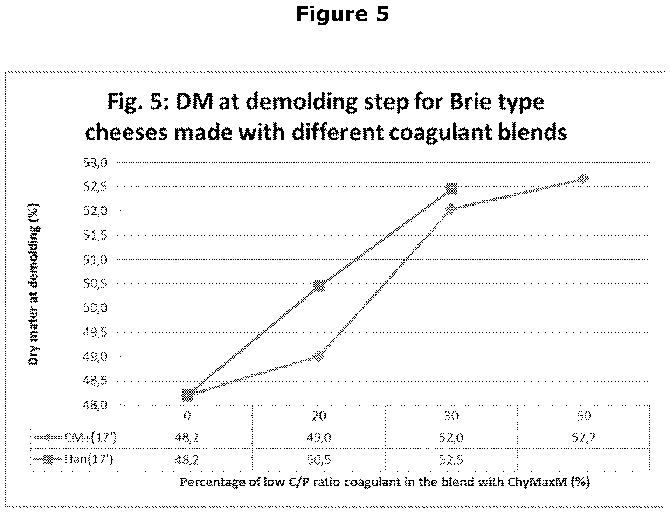

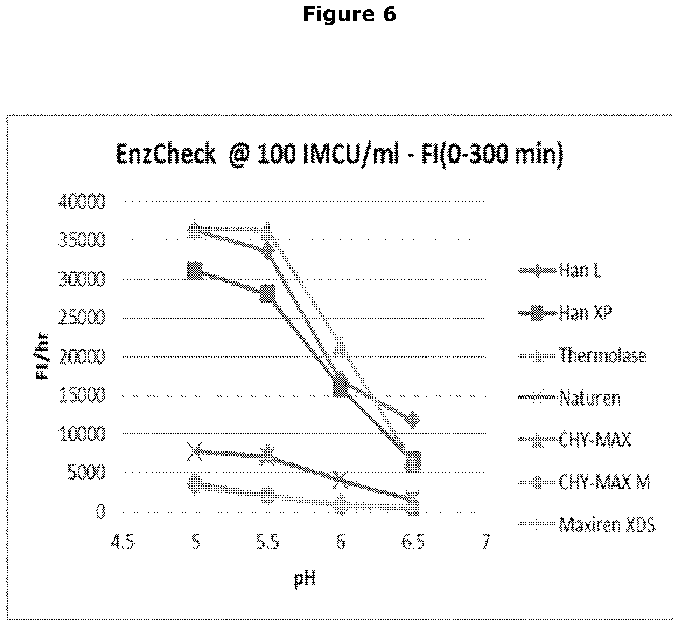

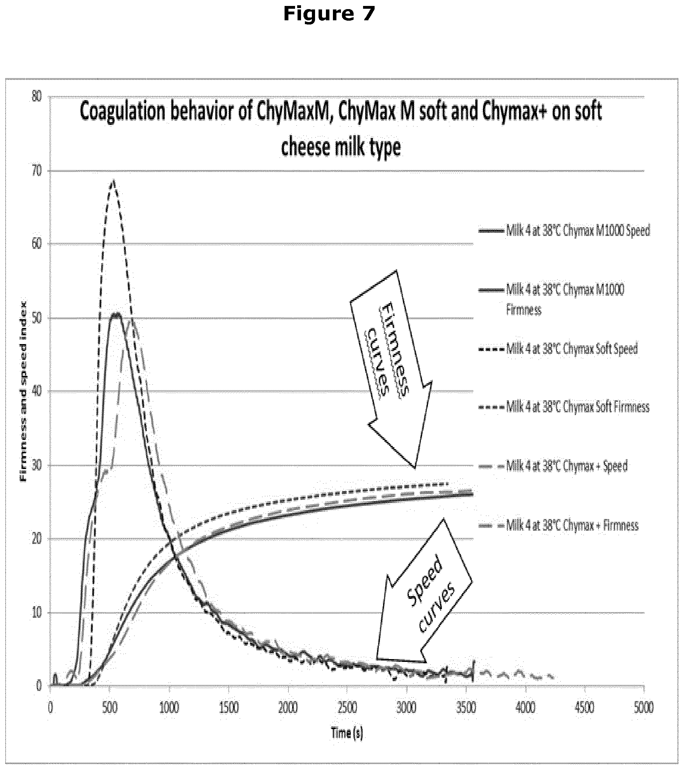

<u>Figure 2</u>: Firmness of brie type cheeses made with different coagulant blends.
<u>Figure 3</u>: Stickiness of brie type cheeses made with different coagulant blends.
<u>Figure 4</u>: The pH at the demolding step for brie type cheeses made with the different coagulant blends.
<u>Figure 5</u>: Dry matter (DM) at the demolding step for brie type cheeses made with the different coagulant blends.
<u>Figure 6</u>: Relative proteolysis specificity of a selection of coagulants of particular interest in present invention.
<u>Figure 7</u>: Coagulation speed and firmness development of three different coagulant solutions: Camel chymosin (Chymax® M1000) marked in red full line, bovine chymosin (Chymax® +) marked in green broken lines and a camel/bovine chymosin blend in a 80/20 ratio (Chymax® soft) marked in blue dotted line.

**DETAILED DESCRIPTION OF THE INVENTION**

As exemplified in detail herein, the present invention relates to compositions comprising two coagulants with different C/P ratios, with different origins; 80 w/w % of one coagulant having a specific milk clotting activity/proteolytic activity ratio of more than 5 IMCU/mU derived from camel and 20 w/w % of a second coagulant having a specific milk clotting activity/proteolytic activity ratio in the range of 0.05 to 1.5IMCU/mU derived from Mucor or derived from bovine; wherein the w/w % indicates the concentration of one coagulant relative to the total amount of coagulant wherein the coagulant derived from camel is a camel chymosin.

In a related aspect, the present invention relates to a composition as disclosed herein, wherein the two coagulants have different C/P ratios and/or clotting activities, such as e.g. at least a three-fold difference.

In a related aspect, milk clotting activity is determined from the time needed for a visible flocculation of a standard milk substrate prepared from a low-heat, low fat milk powder with a calcium chloride solution of 0.5 g per liter (pH ≈ 6.5). The clotting time of a rennet sample is compared to that of a reference standard having known milk-clotting activity and having the same enzyme composition by IDF Standard 110B as the sample. Samples and reference standards are measured under identical chemical and physical conditions. Variant samples are adjusted to approximately 3 IMCU/ml using an 84 mM acetic acid pH 5.5 buffer. Hereafter, 200 μl enzyme is added to 10 ml preheated milk (32°C) in a glass test tube placed in a water bath, capable of maintaining a constant temperature of 32°C $\pm$ 1°C under constant stirring.

The total milk-clotting activity (strength) of a rennet is calculated in International Milk-Clotting Units (IMCU) per ml relative to a standard having the same enzyme composition as the sample according to the formula:

$$\text{Strength in IMCU/ml} = \frac{\text{Sstandard x Tstandard x Dsample}}{\text{Dstandard x Tsample}}$$

Sstandard: The milk-clotting activity of the international reference standard for rennet.
Tstandard: Clotting time in seconds obtained for the standard dilution.
Dsample: Dilution factor for the sample
Dstandard: Dilution factor for the standard
Tsample: Clotting time in seconds obtained for the diluted rennet sample from addition of enzyme to time of flocculation

Determination of total protein content

Total protein content was determined using the Pierce BCA Protein Assay Kit from Thermo Scientific following the instructions of the providers.

Calculation of specific clotting activity

Specific clotting activity (IMCU/mg total protein) was determined by dividing the clotting activity (IMCU/ml) by the total protein content (mg total protein per ml).

In working Example 3 herein is provided an Example of a standard method to determine proteolytical activity.

As an example, general proteolytic activity may be measured using fluoresecently labelled Bodipy-FL casein as a substrate (EnzChek; Molecular Bioprobes, E6638). Casein derivatives heavily labeled with pH-insensitive green-fluorescent Bodipy-FL result in almost complete quenching of the conjugate's fluorescence. Protease catalyzed hydrolysis releases fluorescent Bodipy-FL. This method is very sensitive which is essential for this experiment as high C/P coagulants often have low general proteolytical activity compared to low C/P coagulants.

The assay was conducted in a 0.2 M phosphate buffer adjusted to the desired pH at a final substrate concentration of 0.04 mg/ml. Prior to mixing 1 part of substrate with 1 part of enzyme, both prepared in the phosphate buffer, all enzyme variants where normalized to 50 IMCU/ml (according to example 2). The substrate and enzyme were mixed in a 96-well Nunc Fluoro microtitter plates, sealed and incubated at 32°C for 60 min. After incubation the sealing was removed and the fluorescence recorded in a fluorimeter.

As known in the art - the herein relevant so-called C/P ratio is determined by dividing the specific clotting activity (C)(IMCU/ml) with the proteolytical activity (P)(mU/ml).

As known in the art - a higher C/P ratio implies generally that the loss of protein during e.g. cheese manufacturing due to non-specific protein degradation is reduced, i.e. the yield of cheese is improved, and that the development of bitter taste in the cheese during maturation is reduced.

Another aspect of the invention relates to compositions comprising two or more different chymosins, wherein the compositions entail beneficial properties on the curd firmness and coagulation speed.

One way of measuring this is to use the CHYMOgraph®. The CHYMOgraph® allows for an estimation of the milk flocculation time as well as the curd firmness evolution in cheese production.

The software measures the organization speed of the protein network in the curd. The variation in milk density and curd visco-elasticity is tranformed in user-friendly graphical data, which can be used for the coagulant characterization or to define the optimal curd cut time.

The CHYMOgraph® measures the milk flocculation time, the curd firmness and its evolution and the organisation speed of the protein network.

For preparation of the milk, a container 500 g of milk according to the target of milk composition was prepared and heated to renneting temperature at 38°C in water bath 1 hour before adding rennet. Before use of milk, pH was controlled and register. An example of a preferred milk composition is:

Fortified milk with 3.8% of protein, high heat treated at 90°C for 30s,
pH at renneting: 6.18 at 38°C and 6.28 at (4°C).
Renneting temperature: 38°C. This milk type corresponds to the milk composition used when producing soft cheese milk type.

Preferred coagulant are prepared as: 3 coagulant solutions are tested: Camel chymosin (ChyMax® M1000), Bovine Chymosin (ChyMax®+) and ChyMaxM soft (a blend of 80% Camel chymosin (ChyMax® M1000) and 20% bovine chymosin (ChyMax® +)). Each coagulant solution may be prepared with a strength of 20 IMCU/mL dilution with warm water. This dilution aims to bring enough coagulant in the milk sample to facilitate its dispersion.

Each coagulant solution may be added to the milk (1 mL for 500 g of milk, so 40 IMCU/1L). After coagulant addition the sample was mixed 30 seconds by turning. After turning, 10 mL of each coagulant solution was added to the Chymograph.

As known in the art - different natural wildtype chymosin polypeptide sequences obtained from different mammalian species (such as e.g. bovines, camels, sheep, pigs, or rats) are having a relatively high sequence similarity/identity.

In figure 1 herein this is exemplified by an alignment of herein relevant different chymosin sequences.

In the present context - a naturally obtained wildtype chymosin (such as bovine chymosin or camel chymosin) as shown in figure 1 may herein be an example of a parent polypeptide - i.e. a parent polypeptide to which an alteration is made to produce a variant chymosin polypeptide of the present invention.

In a further related aspect not forming part of the present invention, the present disclosure relates to a composition as disclosed herein, wherein the two or more coagulants form oS1 and/or β-casein at different rates.

In yet a related aspect not forming part of the present invention, the present disclosure relates to a composition as disclosed herein, wherein the two or more coagulants have different general proteolytic activity, such as e.g. at least a three-fold difference.

Hence in an aspect, the present invention provides compositions as described above, wherein the composition comprises at least one coagulant having a C/P ratio of more than 5 IMCU/mU and at least a second coagulant having a C/P ratio in the range of 0.05 to 1.5 IMCU/mU.

For example, the composition may comprise at least 50 w/w % of one coagulant having a C/P ratio of more than 5 IMCU/mU and at least 15 w/w % of a second coagulant having a C/P ratio in the range of 0.05 to 1.5 IMCU/mU, wherein the w/w % indicates the concentration of one coagulant relative to the total amount of coagulant. In a further aspect not forming part of the present invention, the disclosure provides a composition comprising at least 70 w/w % of one coagulant having a C/P ratio of more than 5 IMCU/mU and between 15 and 30 w/w % of a second coagulant having a C/P ratio in the range of 0.05 to 1.5 IMCU/mU, wherein the w/w % indicates the concentration of one coagulant relative to the total amount of coagulant.

In yet a related aspect, the present invention relates to a composition as disclosed herein, wherein the composition comprising a blend of two coagulants derived from two or more different animal or microbial species or genuses.

In yet a related aspect not forming part of the present invention, the present disclosure relates to a composition as disclosed herein, wherein the composition comprises a blend of two or more coagulants that are variants of the same parent coagulant.

In yet a related aspect not forming part of the present invention, the present disclosure relates to a composition as disclosed herein, wherein the two or more different genuses or species is selected from the list consisting of: cow, buffalo, camel, pig, rat, sheep or mucor.

In yet a related aspect not forming part of the present invention, the present disclosure relates to a composition as disclosed herein, wherein at least one of the two or more different coagulants is derived from a coagulant derived from cow, buffalo, camel, pig, rat, sheep and/or mucor.

In yet a related aspect, the present invention relates to a composition as disclosed herein, wherein the composition comprises milk such as e.g. soya milk, sheep milk, goat milk, buffalo milk, yak milk, lama milk, camel milk or cow milk; and

In yet a related aspect, the present invention relates to a composition as disclosed herein, wherein the composition is a blend of a bovine coagulant and a camel coagulant.

In yet a related aspect, the present invention relates to a composition as disclosed herein, wherein the composition comprise from 10-50%, such as e.g. 20% of a non-bovine or a non-camel coagulant, relative to the total amount of coagulant.

In yet a related aspect, the present invention relates to a composition as disclosed herein, wherein the composition comprise a high C/P ratio coagulant and a low C/P ratio coagulant. In yet a related aspect, the present invention relates to a composition as disclosed herein, wherein the composition comprise 20% of a low C/P ratio coagulant relative to the total amount of coagulant.

In a further embodiment the present disclosure not forming part of the present invention, provides a food or feed composition comprising a blend of two or more coagulants as described above.

The food or feed composition can be a soft-cheese, such as e.g. brie or camembert.

In yet a related aspect, the present invention relates to a composition as disclosed herein, wherein the composition is for making soft-cheese, such as e.g. brie or camembert, optionally, the soft-cheese is a surface ripened soft-cheese.

Further, the invention relates to the use of a composition according as described in any of the aspects herein, in a process for making soft-cheese. The composition as described in the aspects herein, may be added no later than 20 minutes, such as e.g. no later than 15 minutes, such as e.g. no later than 10 minutes, such as e.g. no later than 5 minutes after the addition of one or more starter cultures

If applicable, further manufacturing steps to obtain the milk based product any be added.

For example, the present invention provides a method for making a fermented milk product comprising the following steps:

(a) adding a starter culture to milk and incubating the culture at a temperature of 28 to 42 °C for at least five minutes;
(b) adding a composition comprising two coagulants with different C/ P ratios, with different origins; 80 w/w % of one coagulant having a specific milk clotting activity/ proteolytic activity ratio of more than 5 IMCU/mU derived from camel and 20 w/w % of a second coagulant having a specific milk clotting activity/proteolytic activity ratio in the range of 0.05 to 1.5IMCU/mU derived from Mucor or derived from bovine; wherein the w/w % indicates the concentration of one coagulant relative to the total amount of coagulant wherein the coagulant derived from camel is a camel chymosin, in a total concentration between 2000 IMCU/100l and 3500 IMCU/100l;
(c) further incubating the culture at a temperature of 28 to 42 °C for at least three hours;
(d) separating the whey to obtain a cheese.

The method can advantageously be used to make soft-cheese, such as e.g. brie or camembert.

Again, the composition as described in any of the aspects herein may as a related embodiment be added no later than 20 minutes, such as e.g. no later than 15 minutes, such as e.g. no later than 10 minutes, such as e.g. no later than 5 minutes after the addition of one or more starter cultures.

Determining the amino acid position of a chymosin of interest

As discussed above - as a reference sequence for determining the amino acid position of a herein relevant chymosin polypeptide of interest (e.g. camel, sheep, bovine etc.) is herein used the public known bovine chymosin sequence disclosed as SEQ ID NO: 1 herein.

By using well-known computer programs as mentioned above - it is routine work for the skilled person to determine the amino acid position of a herein relevant chymosin polypeptide of interest (e.g. camel, sheep, bovine etc.) in a corresponding chymosin.

A method for making an isolated chymosin polypeptide variant

As discussed above - as known in the art, the skilled person may, based on his common general knowledge, routinely produce and purify chymosin and chymosin variants. Said in other words, once the skilled person is in possession of a herein relevant parent polypeptide having chymosin activity of interest (e.g. from bovines, camels, sheep, pigs, or rats) it is routine work for the skilled person to make a variant of such a parent chymosin of interest.

An example of a suitable method to produce and isolate a chymosin (variant or parent) may be by well-known e.g. fungal recombinant expression/production based technology as e.g. described in WO02/36752A2 (Chr. Hansen).

As known in the art - chymosin activity may be determined by the so-called C/P ratio, which is determined by dividing the specific clotting activity (C) with the proteolytical activity (P).

In working Example 2 herein is described a suitable method to determine the specific clotting activity (C) and in working Example 3 herein is described a suitable method to determine proteolytical activity (P).

As discussed above - as a reference sequence for determining the amino acid position of a herein relevant chymosin polypeptide of interest (e.g. camel, sheep, bovine etc.) is herein used the public known bovine chymosin sequence disclosed as SEQ ID NO: 1 herein.

As discussed above - based on e.g. the computer sequence alignment programs discussed herein - it is routine work for the skilled person to determine the herein relevant amino acid position of a herein relevant chymosin polypeptide of interest (e.g. camel, sheep, bovine etc.).

As understood by the skilled person in the present context - a herein relevant parent polypeptide having chymosin activity may already e.g. be a variant of e.g. a corresponding wildtype chymosin. For instance, a camel chymosin variant with e.g. 5-10 alterations (e.g. substitutions) as compared to wildtype camel chymosin polypeptide of SEQ ID NO: 2 will still be a parent polypeptide that has at least 65% sequence identity with the mature polypeptide of SEQ ID NO: 1 (Bovine) as required in e.g. first aspect herein.

Said in other words, a herein relevant isolated chymosin polypeptide variant may comprise alterations (e.g. substitutions) in other position than the positions of e.g. the first aspect herein.

An embodiment relates to an isolated chymosin polypeptide variant, wherein the alteration comprises a substitution, a deletion or an insertion in at least one amino acid position corresponding to any of positions of e.g. the first aspect herein.

It may be preferred that the difference in chymosins exist in at least one alteration of the peptide is a substitution - i.e. a herein relevant coagulant relates to an isolated bovine chymosin polypeptide variant, such as e.g. a variant wherein the alteration is comprising a substitution in at least one amino acid position corresponding to any of positions 51 and/or 221, preferably A51V and K221M in a mature bovine chymosin parent sequence.

Preferably, the parent polypeptide has at least 70% sequence identity with the mature polypeptide of SEQ ID NO: 1 (bovine chymosin), more preferably the parent polypeptide has at least 75% sequence identity with the mature polypeptide of SEQ ID NO: 1 (bovine chymosin).

As an example - a herein suitable relevant parent polypeptide could e.g. be bovine chymosin A - as known in the art bovine chymosin A may only have one amino acid difference as compared to bovine chymosin B of SEQ ID NO: 1 herein.

As discussed above - in working examples herein were made variants using the polypeptide of SEQ ID NO: 1 (Bovine) as parent polypeptide - such variant may herein be termed bovine chymosin variants.

Accordingly, in a preferred embodiment - the parent polypeptide has at least 90% sequence identity with the mature polypeptide of SEQ ID NO: 1 (bovine chymosin), more preferably the parent polypeptide has at least 95% sequence identity with the mature polypeptide of SEQ ID NO: 1 (bovine chymosin) and even more preferably the parent polypeptide has at least 97% sequence identity with the mature polypeptide of SEQ ID NO: 1 (bovine chymosin). It may be preferred that the parent polypeptide is the mature polypeptide of SEQ ID NO: 1 (bovine chymosin).

As understood by the skilled person in the present context - a herein relevant parent polypeptide having chymosin activity may already e.g. be a variant of e.g. a corresponding wildtype chymosin.

A method for making a milk based product

As discussed above - a composition comprising two or more different coagulants as described herein may be used according to the art - e.g. to make a milk based product of interest (such as e.g. a cheese product).

As discussed above - an aspect of the invention relates to a method for making a food or feed product comprising adding an effective amount of the coagulant blends as described herein to the food or feed ingredient(s) and carrying out further manufacturing steps to obtain the food or feed product.

Preferably, the food or feed product is a milk based product and wherein the method comprises adding an effective amount of the coagulant blend as described herein to milk and carrying out further manufacturing steps to obtain the milk based product.

The milk may e.g. be soy milk, sheep milk, goat milk, buffalo milk, yak milk, lama milk, camel milk or cow milk.

The milk based product may e.g. be a fermented milk product, a quark or a cheese.

**EXAMPLES**

**EXAMPLE 1: alignment and numbering of chymosin protein sequences and variant sequences**

Chymosin protein sequences were aligned using the ClustalW algorithm as provided by the EBI (EBI, tools, multiple sequence alignment, CLUSTALW", http://www.ebi.ac.uk/Tools/msa/clustalw2/) and as described in Larkin MA, Blackshields G, Brown NP, Chenna R, McGettigan PA, McWilliam H, Valentin F, Wallace IM, Wilm A, Lopez R, Thompson JD, Gibson TJ, Higgins DG (2007). Bioinformatics 23(21), 2947-2948.

ClustalW2 settings for multiple sequence alignments were Protein weight Matrix = BLOSUM, GAP open = 10, GAP EXTENSION= 0.05, GAP DISTANCES = 8, No End Gaps, ITERATION = none, NUMITER = 1, CLUSTERING = NJ As a reference sequence the bovine chymosin B preprochymosin was used (Genbank accession number P00794 - disclosed herein as SEQ ID NO: 1), where the N-terminal Methionin has number 1 (MRCL......) and the C-terminal Isoleucin (in the protein sequence ...LAKAI) has number 381. Variants were aligned against the bovine B pre-pro-chymosin and residues were numbered according to the corresponding bovine chymosin residue.

**EXAMPLE 2: Determination of specific chymosin activity**

2.1 Determination of clotting activity

Milk clotting activity was determined using the REMCAT method, which is the standard method developed by the International Dairy Federation (IDF method)

Milk clotting activity is determined from the time needed for a visible flocculation of a standard milk substrate prepared from a low-heat, low fat milk powder with a calcium chloride solution of 0.5 g per liter (pH $\approx$ 6.5). The clotting time of a rennet sample is compared to that of a reference standard having known milk-clotting activity and having the same enzyme composition by IDF Standard 110B as the sample. Samples and reference standards were measured under identical chemical and physical conditions. Variant samples were adjusted to approximately 3 IMCU/ml using an 84 mM acetic acid pH 5.5 buffer. Hereafter, 200 $\mu$l enzyme was added to 10 ml preheated milk (32°C) in a glass test tube placed in a water bath, capable of maintaining a constant temperature of 32°C $\pm$ 1°C under constant stirring.

The total milk-clotting activity (strength) of a rennet was calculated in International Milk-Clotting Units (IMCU) per ml relative to a standard having the same enzyme composition as the sample according to the formula:

$$\text{Strength in IMCU/ml} = \frac{\text{Sstandard x Tstandard x Dsample}}{\text{Dstandard x Tsample}}$$

Sstandard:    The milk-clotting activity of the international reference standard for rennet.

Tstandard:    Clotting time in seconds obtained for the standard dilution.

Dsample:    Dilution factor for the sample

Dstandard:   Dilution factor for the standard

Tsample:    Clotting time in seconds obtained for the diluted rennet sample from addition of enzyme to time of flocculation

2.2 Determination of total protein content

Total protein content was determined using the Pierce BCA Protein Assay Kit from Thermo Scientific following the instructions of the providers.

2.3 calculation of specific clotting activity

Specific clotting activity (IMCU/mg total protein) was determined by dividing the clotting activity (IMCU/ml) by the total protein content (mg total protein per ml).

### EXAMPLE 3: Determination of proteolytic activity

General proteolytic activity was measured using fluoresecently labelled Bodipy-FL casein as a substrate (EnzChek; Molecular Bioprobes, E6638). Casein derivatives heavily labeled with pH-insensitive green-fluorescent Bodipy-FL result in almost complete quenching of the conjugate's fluorescence. Protease catalyzed hydrolysis releases fluorescent Bodipy-FL. This method is very sensitive which was essential for this experiment as CHYMAX M has the lowest general proteolytical activity of all coagulants known to date.

The assay was conducted in a 0.2 M phosphate buffer adjusted to the desired pH at a final substrate concentration of 0.04 mg/ml. Prior to mixing 1 part of substrate with 1 part of enzyme, both prepared in the phosphate buffer, all enzyme variants where normalized to 50 IMCU/ml (according to example 2). The substrate and enzyme were mixed in a 96-well Nunc Fluoro microtitter plates, sealed and incubated at 32°C for 60 min. After incubation the sealing was removed and the fluorescence recorded in a fluorimeter.

### EXAMPLE 4: Evaluation of two types of coagulant blends: camel - bovine chymosin blend and Camel - Mucor miehei coagulant

In soft cheese technology, using a high C/P ratio coagulant such as ChyMax®M has several interests. Some of the more important are: increased shelf life, reduced bitterness, increased fat recovery due to higher firmness at cutting, reduced post-acidification especially in the case of use of fast strains.

However, a general modification which can in some case be considered as a default is higher cheese firmness.

In order to keep the interests of High C/P ratio such as ChyMax®M and reduce firmness of the cheese, a blend of ChyMax®M and other coagulants with lower C/P ratio have been defined.

Brie type stabilized soft cheeses have been produced with different coagulant ratios. Cheeses have been analyzed at demolding and during shelf life.

Milk: (same for all trials)

- Proteins: 36.2g/L (no whey protein where added)
- Fat: 61.7g/L
- Fat/Protein ratio: 1.7
- Pasteurization: 72°C/20s
- Total volume/trial: 500 L (5 vats of 100L)
- CaCl2 added: 15g/100L
- Temperature at coagulant addition: 40°C
- pH at coagulant addition: 6.3
- Coagulant dose: 3000 IMCU/100L of milk (corresponding to 8287 IMCU/g of proteins).

Coagulant blends:

- A first type of blends have been tested: ChyMax®M with 20%, 30% or 50% of ChyMax® (bovine coagulant with low C/P), indicated with red lines with square markers in figures 2-5.
- A second type of blends have been tested: ChyMax®M with 20% or 30% of Hannilase® (*Mucor miehei* coagulant with a low C/P ratio), indicated with blue lines with diamond shaped markers in figures 2-5.

- A Pure ChyMax®M test with the same dosages and parameters has been made as a reference. This Reference is used for both types of blends being a blend with 0% of the non-ChyMax®M component, indicated in the first column of the tables included in figures 2-5.

Results:

(Values are average of 5 vats)

- pH at demolding (fig. 4): a slight tendency to get a lower pH at demolding when increasing the low C/P ratio component is observed for both types of low C/P ratio coagulant used: from pH 5.06 for the pure ChyMax®M test, the pH was 5.03 for blend with 20% Hannilase® and still 5.06 with 20% of ChyMax®. Being close to pH meter precision, this variability is still significant as it is an average of 5 vats. Then at 30%, pH is 5.03 for blends with Hannilase® - similar to 20% blend - and 5.02 for blends with ChyMax® - similar to results with Hannilase® 20% and 30% blends. Then with the 50% ChyMax® blend, the pH is similar to 30% blend. This shows that using a blend of high C/P ratio coagulant such as ChyMax®M and lower C/P ratio such as ChyMax® or Hannilase® has a tendency to reduce pH value at demolding in a brie type soft cheese. This tendency is already effective with 20% of Hannilase when for ChyMax® this tendency is seen only with more than 30%.
- Texture: Texture measurements have been performed on cheeses after 25 days of shelf life using TAXT2 penetrometer with a guillotine tool.
- Firmness (Fig. 2): Firmness was clearly decreased with increasing percentage of low C/P coagulant in the blends and then stabilized for higher percentages. For ChyMax® blends, firmness is decreasing from 1325g for the pure ChyMax®M test to 1180g for the 20% blend then to 1022g for the 30% blend. For Hannilase® blends, firmness is already decreased to 1050 with 20% and then keeps approximately the same value - 1053g - with 30% blend.
- Stickiness (Fig. 3): Stickiness clearly increasing when blending ChyMax®M with a lower C/P ratio coagulant. For 20% both types of low C/P ratio coagulants where slightly increasing stickiness: from a value of 50g/s for the pure ChyMax®M the test with 20% ChyMax® reached 72g/s and the test with 20% Hannilase® blend reached 59g/s. These values corresponded to still acceptable stickiness of the cheeses. Then with 30% both types of blends are reaching values close to 95 (96 for ChyMax® and 92 for Hannilase®). These values where clearly linked with not acceptable cheeses in term of stickiness.
- Texture optimum: The objective of this experiment was to decrease firmness; however the stickiness is not a desired characteristic. Therefore the optimum blends for texture improvement was 20% for both types of low C/P ratio coagulants.
- Dry matter (DM) at demolding (fig. 5): Dry matter is increased with blends compared with the pure ChyMax®M test. For both types of blends the dry mater at demolding is increased at 20% and at 30% - from 48.2% for the pure ChyMax®M test, with 20% blends the dry mater was 49% for ChyMax® blend and 50.5% for Hannilase® blend and for 30% test, dry matter was 52% for ChyMax® blend and 52.5% for Hannilase® blend.
- Dry matter versus firmness: Comparing these dry mater results with texture measurement we can see that even if the cheeses are dryer at demolding with blends compared with pure ChyMax®M trials, the texture is still softer. This shows that blending ChyMax®M with lower C/P coagulants is strongly effective to reduce firmness even with an increased dry matter.
- Optimum results: Using these different results we can see that for a blend of ChyMax®M and ChyMax® as well as for a blend of ChyMax®M and Hannilase®, the optimum ratio is close to 20% to keep the advantages of high C/P ratio coagulant and reduce firmness when this is not wanted.

**EXAMPLE 5: Determination of the coagulation behavior of bovine, camel and blended chymosins.**

To follow the coagulation behavior, we have used the CHYMOgraph® (patented Chr. Hansen tool). The CHYMOgraph® allows for an estimation of the milk flocculation time as well as the curd firmness evolution in cheese production.
The software measures the organization speed of the protein network in the curd. The variation in milk density and curd visco-elasticity is tranformed in user-friendly graphical data, which can be used for the coagulant characterization or to define the optimal curd cut time.
The CHYMOgraph® measures the milk flocculation time, the curd firmness and its evolution and the organisation speed of the protein network.

Milk preparation

A container 500 g of milk according to the target of milk composition was prepared and heated to renneting temperature at 38°C in water bath 1 hour before adding rennet. Before use of milk, pH was controlled and register.

Milk composition

Fortified milk with 3.8% of protein, high heat treatment: 90°C for 30s,
pH at renneting: 6.18 at 38°C and 6.28 at (4°C).
Renneting temperature: 38°C. This milk type corresponds to the milk composition used when producing soft cheese milk type.

Coagulant preparation

3 coagulant solutions were tested: Camel chymosin (ChyMax® M1000), Bovine Chymosin (ChyMax®+) and ChyMaxM soft (a blend of 80% Camel chymosin (ChyMax® M1000) and 20% bovine chymosin (ChyMax® +)). Each coagulant solution was prepared with a strength of 20 IMCU/mL dilution with warm water. This dilution aims to bring enough coagulant in the milk sample to facilitate its dispersion.

Each coagulant solution was added to the milk (1 mL for 500 g of milk, so 40 IMCU/1L). After coagulant addition the sample was mixed 30 seconds by turning. After turning, 10 mL of each coagulant solution was added to the Chymograph.

Results

As depicted in Figure 7, the speed of coagulation when using a blend of bovine and camel chymosin (ChyMax soft) exceeds the speed of coagulation when using camel chymosin (ChyMax® M1000) or bovine chymosin (ChyMax+) alone. This is highly surprising as the skilled scientist would expect a performance of the blend corresponding to the weighted average of the two components included in the blend.

**REFERENCES**

1: WO02/36752A2 (Chr. Hansen)
2: Suzuki et al: Site directed mutagenesis reveals functional contribution of Thr218, Lys220 and Asp304 in chymosin, Protein Engineering, vol. 4, January 1990, pages 69-71
3: Suzuki et al: Alteration of catalytic properties of chymosin by site-directed mutagenesis, Protein Engineering, vol. 2, May 1989, pages 563-569
4: van den Brink et al: Increased production of chymosin by glycosylation, Journal of biotechnology, vol. 125, September 2006, pages 304-310.
5: Pitts et al: Expression and characterisation of chymosin pH optima mutants produced in Tricoderma reesei, Journal of biotechnology, vol. 28, March 1993, pages 69-83
6: M.G. Williams et al: Mutagenesis, biochemical characterization and X-ray structural analysis of point mutants of bovine chymosin, Protein engineering design and selection, vol. 10, September 1997, pages 991-997
7: Strop et al: Engineering enzyme subsite specificity: preparation, kinetic characterization, and x-ray analysis at 2.0 ANG resolution of Val111phe site mutated calf chymosin, Biochemistry, vol. 29, October 1990, pages 9863-9871
8: Supannee et al: Site-specific mutations of calf chymosin B which influence milk-clotting activity, Food Chemistry, vol. 62, June 1998, pages 133-139
9: Zhang et al: Functional implications of disulfide bond, Cys45-Cys50, in recombinant prochymosin, Biochimica et biophysica acta, vol. 1343, December 1997, pages 278-286.
10: WO2013/174840A1 (Chr. Hansen).
11: WO2013/164479A2 (DSM).

**Claims**

1. A composition for clotting milk, the composition comprising

   two coagulants with different C/P ratios, with different origins;
   80 w/w % of one coagulant having a specific milk clotting activity/proteolytic activity ratio of more than 5 IMCU/mU derived from camel and 20 w/w % of a second coagulant having a specific milk clotting activity/proteolytic activity ratio in the range of 0.05 to 1.5IMCU/mU derived from *Mucor* or derived from bovine;
   wherein the w/w % indicates the concentration of one coagulant relative to the total amount of coagulant
   wherein the coagulant derived from camel is a camel chymosin,
   wherein the specific milk clotting activity (C) and the proteolytic activity (P) are determined as described in the description.

2. The composition according to the preceding claim, wherein the composition comprises milk such as e.g. soya milk, sheep milk, goat milk, buffalo milk, yak milk, lama milk, camel milk or cow milk.

3. The composition according to claim 2, wherein the milk is cows milk.

4. The composition according to claim 2 or 3, wherein the milk contain from about 3% to about 5% protein, such as from 3.5% to 4.5% protein, such as 3.8% protein.

5. The composition according to any of claims 2 to 4, wherein the milk comprises from about 1.5% to 5% fat, such as from 2.5% to 4% fat, such as 3.5% fat.

6. The composition according to any of claims 1 to 5, wherein the composition is a blend of camel and bovine chymosin.

7. Use of a composition according to any one of claims 1 to 6 in a process for making soft-cheese.

8. Use according to claim 5, wherein the composition according to any of claims 1 to 6 is added to milk no later than 20 minutes, such as e.g. no later than 15 minutes, such as e.g. no later than 10 minutes, such as e.g. no later than 5 minutes after the addition of one or more starter cultures.

9. A method for making a milk-based product comprising adding an effective amount of the composition according to any of claims 1 to 6 and carrying out further manufacturing steps to obtain the milk based product.

10. A method for making a fermented milk product comprising the following steps:

    (a) adding a starter culture to milk and incubating the culture at a temperature of 28 to 42 °C for at least five minutes;
    (b) adding a composition according to any one of claim 1 to 6 in a total concentration between 2000 IMCU/100I and 3500 IMCU/100I;
    (c) further incubating the culture at a temperature of 28 to 42 °C for at least three hours;
    (d) separating the whey to obtain a cheese.

11. The method according to claim 10, wherein the composition according to any of claims 1 to 6 is added no later than 20 minutes, such as e.g. no later than 15 minutes, such as e.g. no later than 10 minutes after the addition of one or more starter cultures.

12. The method according to claim 10 or 11, wherein the method is used to make soft-cheese, such as e.g. brie or camembert.

**Patentansprüche**

1. Zusammensetzung zur Gerinnung von Milch, wobei die Zusammensetzung Folgendes umfasst

   zwei Koagulationsmittel mit unterschiedlichen C/P-Verhältnissen und unterschiedlicher Herkunft;
   80 Gew.-% eines Koagulationsmittels mit einer spezifischen Milchgerinnungsaktivität/proteolytischen Aktivitätsrate von mehr als 5 IMCU/mU, abgeleitet von Kamel, und 20 Gew.-% eines zweiten Koagulationsmittels mit einer spezifischen Milchgerinnungsaktivität/proteolytischen Aktivitätsrate im Bereich von 0,05 bis 1,5 IMCU/mU, abgeleitet von *Mucor* oder abgeleitet von Rind;

wobei das Gew.-% die Konzentration eines Koagulationsmittels bezogen auf die Gesamtmenge des Koagulationsmittels angibt

wobei das von Kamel abgeleitete Koagulationsmittel ein Kamelchymosin ist,

wobei die spezifische Milchgerinnungsaktivität (C) und die proteolytische Aktivität (P) wie in der Beschreibung beschrieben bestimmt werden.

2. Zusammensetzung nach einem der vorhergehenden Ansprüche, wobei die Zusammensetzung Milch wie z. B. Sojamilch, Schafmilch, Ziegenmilch, Büffelmilch, Yakmilch, Lamamilch, Kamelmilch oder Kuhmilch umfasst.

3. Zusammensetzung nach Anspruch 2, wobei die Milch Kuhmilch ist.

4. Zusammensetzung nach Anspruch 2 oder 3, wobei die Milch etwa 3 % bis etwa 5 % Protein, wie etwa 3,5 % bis 4,5 % Protein, wie etwa 3,8 % Protein, enthält.

5. Zusammensetzung nach einem der Ansprüche 2 bis 4, wobei die Milch etwa 1,5 % bis 5 % Fett, wie etwa 2,5 % bis 4 % Fett, wie etwa 3,5 % Fett, umfasst.

6. Zusammensetzung nach einem der Ansprüche 1 bis 5, wobei die Zusammensetzung eine Mischung aus Kamel- und Rinderchymosin ist.

7. Verwendung einer Zusammensetzung nach einem der Ansprüche 1 bis 6 in einem Verfahren zur Herstellung von Weichkäse.

8. Verwendung nach Anspruch 5, wobei die Zusammensetzung nach einem der Ansprüche 1 bis 6 der Milch spätestens 20 Minuten, wie z. B. spätestens 15 Minuten, wie z. B. spätestens 10 Minuten, wie z. B. spätestens 5 Minuten, nach der Zugabe einer oder mehrerer Starterkulturen zugesetzt wird.

9. Verfahren zum Herstellen eines Produkts auf Milchbasis, umfassend das Zusetzen einer wirksamen Menge der Zusammensetzung nach einem der Ansprüche 1 bis 6 und Durchführen weiterer Herstellungsschritte, um das Produkt auf Milchbasis zu erhalten.

10. Verfahren zum Herstellen eines fermentierten Milchprodukts, umfassend die folgenden Schritte:

(a) Zugeben einer Starterkultur zur Milch und Inkubieren der Kultur bei einer Temperatur von 28 bis 42 °C für mindestens fünf Minuten;
(b) Zugeben einer Zusammensetzung nach einem der Ansprüche 1 bis 6 in einer Gesamtkonzentration zwischen 2000 IMCU/100 L und 3500 IMCU/100 L;
(c) weiteres Inkubieren der Kultur mindestens drei Stunden lang bei einer Temperatur von 28 bis 42 °C;
(d) Abtrennen der Molke, um einen Käse zu erhalten.

11. Verfahren nach Anspruch 10, wobei die Zusammensetzung nach einem der Ansprüche 1 bis 6 spätestens 20 Minuten, wie z. B. spätestens 15 Minuten, wie z. B. spätestens 10 Minuten, nach der Zugabe einer oder mehrerer Starterkulturen zugegeben wird.

12. Verfahren nach Anspruch 10 oder 11, wobei das Verfahren zum Herstellen von Weichkäse, wie z. B. Brie oder Camembert, verwendet wird.

**Revendications**

1. Composition pour le caillage du lait, la composition comprenant

deux coagulants avec des rapports C/P différents, d'origines différentes ;
80 % p/p d'un premier coagulant ayant un rapport activité spécifique de caillage du lait/activité protéolytique supérieur à 5 IMCU/mU issu de camélidé et 20 % p/p d'un second coagulant ayant un rapport activité spécifique

**EP 3 255 997 B1**

de caillage du lait/activité protéolytique dans la plage de 0,05 à 1,5 IMCU/mU issu de *Mucor* ou de bovin ;
dans laquelle le % p/p indique la concentration d'un coagulant par rapport à la quantité totale de coagulant
dans laquelle le coagulant d'origine cameline est une chymosine cameline,
dans laquelle l'activité spécifique de caillage du lait (C) et l'activité protéolytique (P) sont déterminées comme
décrit dans la description.

2. Composition selon la revendication précédente, dans laquelle la composition comprend du lait tel que par exemple du lait de soja, du lait de brebis, du lait de chèvre, du lait de bufflonne, du lait de yack, du lait de lama, du lait de chamelle ou du lait de vache.

3. Composition selon la revendication 2, dans laquelle le lait est du lait de vache.

4. Composition selon la revendication 2 ou 3, dans laquelle le lait contient d'environ 3 % à environ 5 % de protéines, par exemple de 3,5 % à 4,5 % de protéines, par exemple 3,8 % de protéines.

5. Composition selon l'une quelconque des revendications 2 à 4, dans laquelle le lait comprend d'environ 1,5 % à 5 % de matières grasses, par exemple de 2,5 % à 4 % de matières grasses, par exemple 3,5 % de matières grasses.

6. Composition selon l'une quelconque des revendications 1 à 5, dans laquelle la composition est un mélange de chymosine cameline et bovine.

7. Utilisation d'une composition selon l'une quelconque des revendications 1 à 6 dans un procédé de fabrication de fromage à pâte molle.

8. Utilisation selon la revendication 5, dans laquelle la composition selon l'une quelconque des revendications 1 à 6 est ajoutée au lait au plus tard 20 minutes, par exemple au plus tard 15 minutes, par exemple au plus tard 10 minutes, par exemple au plus tard 5 minutes après l'ajout d'un ou plusieurs levains.

9. Procédé de préparation d'un produit à base de lait comprenant l'ajout d'une quantité efficace de la composition selon l'une quelconque des revendications 1 à 6 et la réalisation d'autres étapes de fabrication pour obtenir le produit à base de lait.

10. Procédé de fabrication d'un produit laitier fermenté comprenant les étapes suivantes :

    (a) l'ajout d'un levain au lait et l'incubation la culture à une température de 28 à 42 °C pendant au moins cinq minutes ;
    (b) l'ajout d'une composition selon l'une quelconque des revendications 1 à 6 dans une concentration totale comprise entre 2000 IMCU/100 1 et 3500 IMCU/100 1 ;
    (c) l'incubation supplémentaire de la culture à une température de 28 à 42 °C pendant au moins trois heures ;
    (d) la séparation du lactosérum pour obtenir un fromage.

11. Procédé selon la revendication 10, dans lequel la composition selon l'une quelconque des revendications 1 à 6 est ajoutée au plus tard 20 minutes, par exemple au plus tard 15 minutes, par exemple au plus tard 10 minutes après l'ajout d'un ou plusieurs levains.

12. Procédé selon la revendication 10 ou 11, dans lequel le procédé est utilisé pour fabriquer du fromage à pâte molle, tel que par exemple du brie ou du camembert.

**14**

# Figure 1

```
                         1                                                      50
Bos_bovis_chymosin_B     MRCLVVLLAV FALSQGAEIT RIPLYKGKSL RKALKEHGLL EDFLQKQQYG
             Sheep       MRCLVVLLAV FALSQGAEIT RIPLYKGKPL RKALKERGLL EDFLQKQQYG
       C._bactrianus     MRCLVVLLAA LALSQASGIT RIPLHKGKTL RKALKERGLL EDFLQRQQYA
  Camelus_dromedarius     MRCLVVLLAA LALSQASGIT RIPLHKGKTL RKALKERGLL EDFLQRQQYA
               Pig       .IRGRVLLAV LALSQGSGIT RVPLRKGKSL RKELKERGLL EDFLQKQPYA
               Rat       MRCFVLLLAV LAIAQSHVVT RIPLHKGKSL RNTLKEQGLL EDFLRRHQYE

                         51                                                     100
Bos_bovis_chymosin_B     ISSKYSGFGE VASVPLTNYL DSQYFGKIYL GTPPQEFTVL FDTGSSDFWV
             Sheep       VSSEYSGFGE VASVPLTNYL DSQYFGKIYL GTPPQEFTVL FDTGSSDFWV
       C._bactrianus     VSSKYSSLGK VAREPLTSYL DSQYFGKIYI GTPPQEFTVV FDTGSSDLWV
  Camelus_dromedarius     VSSKYSSLGK VAREPLTSYL DSQYFGKIYI GTPPQEFTVV FDTGSSDLWV
               Pig       LSSKYSSFGE VASEPLTNYL DTQYFGKIYI GTPPQEFTVV FDTGSSELWV
               Rat       FSEKNSNIGM VASEPLTNYL DSEYFGLIYV GTPPQEFKVV FDTGSSELWV

                         101                                                    150
Bos_bovis_chymosin_B     PSIYCKSNAC KNHQRFDPRK SSTFQNLGKP LSIHYGTGSM QGILGYDTVT
             Sheep       PSIYCKSNAC KNHQRFDPRK SSTFQNLGKP LSIRYGTGSM QGILGYDTVT
       C._bactrianus     PSIYCKSNAC KNHHRFDPRK SSTFRNLGKP LSIHYGTGSI EGFLGYDTVT
  Camelus_dromedarius     PSIYCKSNVC KNHHRFDPRK SSTFRNLGKP LSIHYGTGSM EGFLGYDTVT
               Pig       PSVYCKSDAC QNHHRFNPSK SSTFQNLDKP LSIQYGTGSI QGFLGYDTVM
               Rat       PSVYCSSKVC RNHNRFDPSK SFTFQNLSKP LFVQYGTGSV EGFLAYDTVT

                         151                                                    200
Bos_bovis_chymosin_B     VSNIVDIQQT VGLSTQEPGD VFTYAEFDGI LGMAYPSLAS EYSIPVFDNM
             Sheep       VSNIVDIQQT VGLSTQEPGD VFTYAEFDGI LGMAYPSLAS EYSVPVFDNM
       C._bactrianus     VSNIVDPNQT VGLSTEQPGE VFTYSEFDGI LGLAYPSLAS EYSVPVFDNM
  Camelus_dromedarius     VSNIVDPNQT VGLSTEQPGE VFTYSEFDGI LGLAYPSLAS EYSVPVFDNM
               Pig       VAGIVDAHQT VGLSTQEPSD IFTYSEFDGI LGLGYPELAS EYTVPVFDNM
               Rat       VSDIVPHQT VGLSTEEPGD IFTYSPFDGI LGLAYPTFAS KYSVPIFDNM

                         201                                                    250
Bos_bovis_chymosin_B     MNRHLVAQDL FSVYMDRNGQ ESMLTLGAID PSYYTGSLHW VPVTVQQYWQ
             Sheep       MDRRLVAQDL FSVYMDRSGQ GSMLTLGAID PSYYTGSLHW VPVTLQKYWQ
       C._bactrianus     MDRHLVARDL FSVYMDRNGQ GSMLTLGATD PSYYTGSLHW VPVTVQQYWQ
  Camelus_dromedarius     MDRHLVARDL FSVYMDRNGQ GSMLTLGAID PSYYTGSLHW VPVTLQQYWQ
               Pig       MHRHLVAQDL FAVYMSRNDE GSMLTLGAID PSYYTGSLHW VPVTMQLYWQ
               Rat       MNRHLVAQDL FSVYMSRNDQ GSMLTLGAID QSYFIGSLHW VPVTVQGYWQ

                         251                                                    300
Bos_bovis_chymosin_B     FTVDSVTISG VVVACEGGCQ AILDTGTSKL VGPSSDILNI QQAIGATQNQ
             Sheep       FTVDSVTISG AVVACEGGCQ AILDTGTSKL VGPSSDILNI QQAIGATQNQ
       C._bactrianus     VTVDSVTING VAVACVGGCQ AILDTGTSVL FGPSSDILKI QMAIGATENR
  Camelus_dromedarius     FTVDSVTING VAVACVGGCQ AILDTGTSVL FGPSSDILKI QMAIGATENR
               Pig       FTVDSVTING VVVACNGGCQ AILDTGTSML AGPSSDILNI QMAIGATESQ
               Rat       FTVDRITIND EVVACQGGCP AVLDTGTALL TGPGRDILNI QHAIGAVQGQ

                         301                                                    350
Bos_bovis_chymosin_B     YGEFDIDCDN LSYMPTVVFE INGKMYPLTP SAYTSQDQGF CTSGFQSENH
             Sheep       YGEFDIDCDS LSSMPTVVFE INGKMYPLTP YAYTSQEEGF CTSGFQGENH
       C._bactrianus     YGEFDVNCGS LRSMPTVVFE INGRDFPLAP SAYTSKDQGF CTSGFQGDNN
  Camelus_dromedarius     YGEFDVNCGN LRSMPTVVFE INGRDYPLSP SAYTSKDQGF CTSGFQGDNN
               Pig       YGEFDIDCGS LSSMPTVVFE ISGRMYPLPP SAYTNQDQGF CTSGFQGDSK
               Rat       HDQFDIDCWR LNFMPTVVFE INGREFPLPP SAYTNQFQGS CSSGFR..HG

                         351                            381
Bos_bovis_chymosin_B     SQKWILGDVF IREYYSVFDR ANNLVGLAKA I
             Sheep       SHQWILGDVF IREYYSVFDR ANNLVGLAKA I
       C._bactrianus     SELWILGDVF IREYYSVFDR ANNRVGLAKA I
  Camelus_dromedarius     SELWILGDVF IREYYSVFDR ANNRVGLAKA I
               Pig       SQHWILGVVF IQEYYSVFDR ANNRVGLAKA I
               Rat       SQMWILGDVF IREFYSVFDR ANNRVGLAKA I
```

# Figure 2

Fig. 2: Firmness of Bries type cheeses made with different coagulant blends

| | 0 | 20 | 30 | 50 |
|---|---|---|---|---|
| CM+ | 1325 | 1180 | 1022 | 1058 |
| Han | 1325 | 1048 | 1053 | |

Percentage of low C/P ratio coagulant in the blend with ChyMaxM (%)

# Figure 3

Fig. 3: Stickyness of Brie type cheeses made with different coagulant blends

| | 0 | 20 | 30 | 50 |
|---|---|---|---|---|
| CM+ | 50 | 72 | 96 | 90 |
| Han | 50 | 59 | 92 | |

Percentage of LowC/P ratio coagulant in the blend with ChyMaxM (%)

# Figure 4

Fig. 4: pH at demolding step for Brie type cheeses made with different coagulant blends

| | 0 | 20 | 30 | 50 |
|---|---|---|---|---|
| CM+(17') | 5,06 | 5,06 | 5,02 | 5,02 |
| Han(17') | 5,06 | 5,03 | 5,04 | |

Percentage of low C/P ratio coagulant in the blend with ChyMaxM (%)

# Figure 5

Fig. 5: DM at demolding step for Brie type cheeses made with different coagulant blends

| | 0 | 20 | 30 | 50 |
|---|---|---|---|---|
| CM+(17') | 48,2 | 49,0 | 52,0 | 52,7 |
| Han(17') | 48,2 | 50,5 | 52,5 | |

Percentage of low C/P ratio coagulant in the blend with ChyMaxM (%)

# Figure 6

## Figure 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0236752 A2, Chr. Hansen
- WO 2013174840 A1, Chr. Hansen

- WO 2013164479 A2

### Non-patent literature cited in the description

- **SUZUKI et al.** Site directed mutagenesis reveals functional contribution of Thr218, Lys220 and Asp304 in chymosin. *Protein Engineering,* 04 January 1990, 69-71
- **SUZUKI et al.** Alteration of catalytic properties of chymosin by site-directed mutagenesis. *Protein Engineering,* 02 May 1989, 563-569
- **VAN DEN BRINK et al.** Increased production of chymosin by glycosylation. *Journal of biotechnology,* September 2006, vol. 125, 304-310
- **PITTS et al.** Expression and characterisation of chymosin pH optima mutants produced in Tricoderma reesei,. *Journal of biotechnology,* 28 March 1993, 69-83
- **M.G. WILLIAMS et al.** Mutagenesis, biochemical characterization and X-ray structural analysis of point mutants of bovine chymosin. *Protein engineering design and selection,* 10 September 1997, 991-997
- **STROP et al.** Engineering enzyme subsite specificity: preparation, kinetic characterization, and x-ray analysis at 2.0 ANG resolution of Val111phe site mutated calf chymosin. *Biochemistry,* 29 October 1990, 9863-9871
- **SUPANNEE et al.** Site-specific mutations of calf chymosin B which influence milk-clotting activity. *Food Chemistry,* June 1998, vol. 62, 133-139

- **ZHANG et al.** Functional implications of disulfide bond, Cys45-Cys50, in recombinant prochymosin. *Biochimica et biophysica acta,* December 1997, vol. 1343, 278-286
- **LARKIN MA ; BLACKSHIELDS G ; BROWN NP ; CHENNA R ; MCGETTIGAN PA ; MCWILLIAM H ; VALENTIN F ; WALLACE IM ; WILM A ; LOPEZ R.** *Bioinformatics,* 2007, vol. 23 (21), 2947-2948
- **SUZUKI et al.** Site directed mutagenesis reveals functional contribution of Thr218, Lys220 and Asp304 in chymosin. *Protein Engineering,* January 1990, vol. 4, 69-71
- **PITTS et al.** Expression and characterisation of chymosin pH optima mutants produced in Tricoderma reesei. *Journal of biotechnology,* March 1993, vol. 28, 69-83
- **M.G. WILLIAMS et al.** Mutagenesis, biochemical characterization and X-ray structural analysis of point mutants of bovine chymosin. *Protein engineering design and selection,* September 1997, vol. 10, 991-997
- **STROP et al.** Engineering enzyme subsite specificity: preparation, kinetic characterization, and x-ray analysis at 2.0 ANG resolution of Val111phe site mutated calf chymosin. *Biochemistry,* October 1990, vol. 29, 9863-9871